# EUROPEAN PATENT SPECIFICATION

(11) **EP 2 269 615 B1**
(45) Date of publication and mention of the grant of the patent: **07.03.2018**
(21) Application number: 10181232.9
(22) Date of filing: 30.05.2003
(51) Int. Cl.: A61K 33/04, A61Q 17/00, A61Q 19/00, A61K 8/37

(54) **Use of dimethyl sulphone in formulations for chemical peeling**
Verwendung von Dimethylsulfon in Formulierungen zum chemischen Peeling
Utilisation de diméthyl sulphone dans des formulations de décapage chimique

(43) Date of publication of application: 05.01.2011
(62) Divisional of application: 03733016.4
(73) Proprietor: De Paoli Ambrosi, Gianfranco, 25010 San Felice del Benaco (BS) (IT)
(72) Inventor: De Paoli Ambrosi, Gianfranco, 25010 San Felice del Benaco (BS) (IT)
(74) Representative: Rigamonti, Dorotea

(56) References cited:
- EP-A1- 1 304 101
- WO-A1-94/05301
- WO-A1-2004/105722
- WO-A1-2004/105741
- DE-A1- 2 452 119

## Description

### FIELD OF THE INVENTION

the present invention relates to the use of dimethy, sulphone in formulations to be used for carrying out chemical peeling.

### STATE OF THE ART

A very superficial peeling accelerates the natural exfoliation of the corneous layer, whilst a peeling which acts at a much deeper level causes necrosis and inflammation of the epidermis, the papillary dermis or of the reticular dermis.

Chemical peeling produces apparent changes in the skin through three mechanisms of action:
1. The stimulation of cellular turnover through the removal of the dead cells from the corneous layer.
2. The elimination of damaged and degenerated epidermal cells, which will be replaced by normal epidermal cells. This result will be particularly evident in the treatment of actinic keratosis and anomalous pigmentations.
3. The introduction of an inflammatory reaction and the activation of the mediators of inflammation (a mechanism still poorly understood) which activates the production of new collagen fibres and glycosaminoglycans (revitalising mechanisms of the dermis).

Since the peeling agents which operate at deep epidermal levels also carry the risk of complications and undesired outcome, it is indispensable to carry out treatments and therapies which achieve excellent results with the least possible risk.

Chemical peeling is particularly recommended in the following cases:
a) Keratosis and cutaneous ageing
b) Dischromia
c) Post - acne scarring
d) Common acne and rosacea
e) Radiodermatitis
f) Stretch marks
g) Seborrheic dermatitis

The various types of chemical peeling can be classified thus:
- Very superficial peeling: this type of peeling only removes the superficial corneous layer;
- Superficial peeling: this type of peeling causes necrosis of a part or in all of the epidermal layer reaching the basal layer of the epidermis;
- Average depth peeling: this type of peeling causes necrosis of the epidermis and part of the papillary dermis;
- Deep peeling: this type of peeling causes necrosis of the epidermis, the papillary dermis and can extend to the reticular dermis.

For chemical peeling the following chemical substances are generally used:
1. Retinoic acid
2. 5-Fluorouracile (5-Fu)
3. Jessner's Solution
4. Resorcine
5. Salicylic acid
6. Trichloroacetic acid
7. Alpha-hydroxy-acids
8. Alpha-keto-acids (such as for example pyruvic acid)
9. Phenol

The depth of peeling depends on numerous factors, such as: i) the type of substance used; ii) the concentration of the substance used, iii) the number of steps with the chosen substance on the same part of the skin, iv) the application technique, v) the preparation of the skin in the pre-treatment phase, vi) the type of cutaneous treatment in the period preceding the peeling, vii) the patients skin type, viii) the area of the cuteous treated, and ix) the exposure time to the selected chemical agent on the skin.

Considering all these variables, it is easy to understand that any classification relating to the various types of peeling has problems, since with the same substance we can obtain a superficial result on one type of skin, whilst on another type of skin we can obtain a medium or deep peeling. It is therefore not rare to cause even considerable damage to the skin, damage which, due to the variability delineated above, is frequently difficult to foresee.

WO 94/05301 discloses a composition for improving the housewife dermatitis comprising precipitated sulphur, salicylic acid and dimethylsulphone.WO 2004/105741 discloses cosmetic and/or pharmaceutical composition comprising dimethylsulphone.

The problem which lies at the heart of the present invention is therefore that of making available a formulation which allows on the one hand the attainment of an efficacious chemical peeling and which on the other hand minimises the risks of damage to the skin of the treated subject.

Such a problem is solved by a formulation for chemical peeling as delineated in the attached claims.

### DESCRIPTION OF THE INVENTION

It has been surprisingly found that the combination between the keratolytic agent and dimethyl isosorbide allows the attainment of an improvement in the absorption kinetics of the compound with keratolytic action; that leads to the use of lower quantities of keratolytic agent than those normally used despite achieving the same or more efficacious and efficient keratolytic action.

The use of lower quantities of keratolytic agent has as a consequence a drastic reduction of the recognisable side effects in the damage to the epidermis and to the dermis.

The present description refers to dimethyl sulphone combined with a keratolytic agent or a mixture of keratolytic agents for use as anti-inflammatory agent against the inflammation produced by a keratolytic agent or a mixture of keratolytic agents, wherein dimethyl sulphone is present in a quantity comprised between 2% and 70% by weight, preferably between 10% and 60% by weight with respect to the keratolytic agent or a mixture of keratolytic agents. The dimethyl isosorbide will be present in such a quantity as to obtain an increase in the absorption kinetics of said keratolytic agent, if compared with the use of the keratolytic agent by itself. The increase in absorption kinetics is evaluated as shown below, i.e. by determining the quantity of keratolytic agent able to permeate through an SCE membrane by HPLC.

Preferred keratolytic compounds are selected from the chemical group of saturated and unsaturated monocarboxylic acids, saturated and unsaturated bicarboxylic acids, tricarboxylic acids, alpha hydroxyacids and beta hydroxyacids of monocarboxylic acids, alpha hydroxyacids and beta hydroxyacids of bicarboxylic acids, alpha hydroxyacids and beta hydroxyacids of tricarboxylic acids, ketoacids, alpha ketoacids, beta ketoacids, of the polycarboxylic acids, of the polyhydroxy monocarboxylic acids, of the polyhydroxy bicarboxylic acids, of the polyhydroxy tricarboxylic acids. Particularly preferred keratolytic agents are selected from the group comprising glycolic acid, tartaric acid, salicylic acid, citric acid, lactic acid, pyruvic acid, gluconic acid, glucuronic acid, malic acid, oxalic acid, malonic acid, succinic acid, acetic acid, phenol, resorcine, retinoic acid, adapalene, trichloroacetic acid, 5-fluoro uracil, azelaic acid. Keratolytic agents comprised within the scope of the present invention are also the salts, esters, possible cis or trans forms, racemic mixtures and/or the relative dextrorotatory or levorotatory forms of the above listed compounds. Such substances can be used singularly or in associations with each other.

According to a particularly preferred embodiment of the present description the pharmaceutical and/or cosmetic composition of dimethyl sulphone with one or more keratolytic agents comprises additionally dimethyl isosorbid. According to the present invention, the dimethyl sulphone combined with keratolytic agents is capable of reducing the erythema induced by the agents themselves. According to this embodiment, the reduction of inflammation, irritation and erythema is obtained through the combination of the activity of the dimethyl sulphone with the fact that the quantity of keratolytic agent used to obtain the "peeling" effect is reduced thanks to the action of the dimethyl isosorbide. This latter component, as mentioned previously, increases the kinetics of percutaneous absorption of the keratolytic agent, rendering it more available for the action intended.

A third particularly preferred embodiment here described is that in which the keratolytic agent and/or the mixed keratolytic agents, combined with dimethyl isosorbide and dimethyl sulphone, are associated with the ester of an acid with keratolytic activity.

When used in association, the dimethyl isosorbide and the keratolytic agent and/or a mixture of keratolytic agents, can each be contained in the composition in a quantity by weight of from 1 to 99%, preferably each in a quantity comprised of between 5 and 40%. More preferably, dimethyl isosorbide and the keratolytic agents will be present in the composition in weight ratios comprised of between 1 : 4 and 4 : 1.

When used in association, the dimethyl isosorbide and the keratolytic agent and/or a mixture of keratolytic agents, associated with dimethyl sulphone can be contained in the compositions in a quantity by weight of from 1 to 99% each, preferably in a quantity comprised of between 5 and 70%. More preferably, dimethyl isosorbide and the keratolytic agent will be present in the composition in a ratio comprised of between 1 : 4 and 4 : 1. The dimethyl sulphone will preferably be present in a quantity comprised of between 2% and 70% by weight, more preferably between 10% and 65%, with respect to the keratolytic agent.

In the compositions of the description, both based on keratolytic agent/dimethyl isosorbide and keratolytic agent/isosorbide/dimethyl sulphone, the weight balance up to 100% will be attained by the addition of solvents - such as water (in particular demineralised water), alcohols (such as ethyl alcohol) or glycols (for example, ethylene glycol or propylene glycol) - and/or excipients such as emulsifiers, antioxidants, lipid excipients, sequestrants, preservatives. Such excipients, used in particular for the preparation of emulsions, gels, creams, ointments, etc., are widely known to the expert in the field and will therefore not be described in any further detail.

Experiments relating to the evaluation of the favourable effect on percutaneous absorption of the keratolytic agent in the presence of dimethyl isosorbide is reported in corroboration of the present description.

The aim of these experiments has been that of evaluating the *in vitro* percutaneous absorption across isolated human skin, of glycolic acid comprised in a formulation in which the keratolytic agent has been dissolved in water and propylene glycol (solution GC1) and another in which the keratolytic agent has been vehicularised using dimethyl isosorbide (solution GC2) .

The experiment has been carried out using a system of Franz cells with a corneous-epidermis membrane layer (SCE membrane), the experimental protocol of which has already been widely described in the literature.

### Preparation of the SCE membranes

The preparation of the corneous-epidermis membrane layer (SCE) has been carried out using a technique already described in the literature, using samples of human skin originating from subjects, of ages comprised of between 32 and 45 years, subjected to reductive plastic surgery.

In these skin samples, following separation from the subcutaneous adipose layer and immersion in distilled water at a temperature of 60 °C for a few minutes, the dermis has been separated to obtain the SCE membranes used in this study. The removal of the dermis is made necessary because, in the *in vitro* evaluation of the percutaneous absorption of lipophilic substances, this tissue can be a "dummy" and additional barrier with respect to the *in vivo* cutaneous permeation process. The SCE membranes, thus prepared, have been dried and then placed in an appropriate desiccator. These membranes have then been conserved in sheets of aluminium at a temperature of around 4°C and rehydrated at the time of use, by immersion in distilled water, one hour prior to the start of the permeation experiments. Prior to proceeding to the cutaneous permeation experiments and with the aim of evaluating the integrity of the SCE membranes used, the coefficient of permeability (Kp) of tritiated water has been determined for each sample of SCE membranes, the value of which is a sufficiently indicative parameter of the integrity of said membranes.

### Cutaneous permeation experiments

For the evaluation of the degree of *in vitro* percutaneous absorption of glycolic acid from the formulations GC1 and GC2, batteries of six Franz cells (LGA, Berkeley, CA) have been used. Each Franz cell was constituted by a "donor" and a "receptor" between which has been placed the SCE membrane with the corneous layer facing the "donor". The volume of the "receptor" of the cell was 4.7 ml whilst the surface area of the membrane in the "donor" (and therefore the potential cutaneous surface in contact with the product) was 0.75 cm².

The "receptor" compartment, stirred and thermostated at a temperature of 35-36 °C, has been fed with an aqueous saline solution of 0.9% (w/v) NaCl.

For the permeation experiment, 200 mg/cm² of each formulation GC1 and GC2, containing the glycolic acid, have been initially deposited onto each SCE membrane. The monitoring of the permeation process has then been carried out by determining, by a suitable HPLC method, the quantity of glycolic acid that has permeated through the corneous-epidermis (SCE) membrane layer over the 24 hour period following the application of the product into the "donor". In order to carry out this experiment samples of SCE membranes originating from six different subjects (n=6) have been used, whilst each single permeation experiment has been performed in duplicate. The results have been expressed as the quantity of permeated glycolic acid, per cm² of skin, in 24 hours.

### HPLC determinations

The determinations of the quantities of glycolic acid, present in the receiver phase of the Franz cell 24 hours after the application of the formulations GC1 and GC2 have been carried out using an appropriate HPLC method reported in the literature.

### Results

The results obtained (see Tab. 1) in the studies of the cutaneous permeation of glycolic acid from the formulations GC1 and GC2, demonstrate that the GC2 formulation is able to double (p < 0.01) the quantity of glycolic acid permeated across the SCE membranes with respect to the GC1 formulation.

Tab. 1 - The quantity of glycolic acid (expressed in µg/cm²) permeated from the formulations GC1 and GC2 across the human skin (SCE) membranes originating from six different subjects (A-F) in 24 hours.

| Subject | CG1 | CG2 |
|---|---|---|
| A | 65.7 | 143.8 |
| B | 82.7 | 169.2 |
| C | 39.4 | 95.3 |
| D | 75.1 | 95.2 |
| E | 112.5 | 197.3 |
| F | 76.1 | 235.4 |
| Mean | 75.2 | 156.0 |
| ± | 23.7 | 56.1 |

In the following are herein reported some examples of formulations according to the present description.

### EXAMPLES of formulations

### Preparation 1 as comparative example

| N° | Description | % (w/w) a |
|---|---|---|
| 01 | Dimethyl isosorbide | 50.00 |
| 02 | Pyruvic acid | 50.00 |

Method of preparation: mix 01 and 02

### Preparation 2

| N° | Description | % (w/w) a |
|---|---|---|
| 01 | Dimethyl isosorbide | 40.00 |
| 02 | Pyruvic acid | 50.00 |
| 03 | Dimethyl sulphone | 10.00 |

Method of preparation: dissolve 03 in 01, to the solution obtained, mix in 02

### Preparation 3 as comparative example

| N° | Description | % (w/w) a |
|---|---|---|
| 01 | Dimethyl isosorbide | 50.00 |
| 02 | Acetic acid | 50.00 |

Method of preparation: mix 01 and 02

### Preparation 4

| N° | Description | % (w/w) a |
|---|---|---|
| 01 | Dimethyl isosorbide | 40.00 |
| 02 | Acetic acid | 50.00 |
| 03 | Dimethyl sulphone | 10.00 |

Method of preparation: dissolve 03 in 01, to the solution obtained, mix in 02

### Preparation 5 as comparative example

| N° | Description | % (w/w) a |
|---|---|---|
| 01 | Dimethyl isosorbide | 75.00 |
| 02 | Trichloroacetic acid | 25.00 |

Method of preparation: mix 02 in 01

### Preparation 6

| N° | Description | % (w/w) a |
|---|---|---|
| 01 | Dimethyl isosorbide | 75,00 |
| 02 | Trichloroacetic acid | 15,00 |
| 03 | Dimethyl sulphone | 10,00 |

Method of preparation: dissolve 03 in 01, to the solution obtained, mix in 02

### Preparation 7 as comparative example

| N° | Description | % (w/w) a |
|---|---|---|
| 01 | Dimethyl isosorbide | 75.00 |
| 02 | Salicylic acid | 25.00 |

Method of preparation: mix 02 in 01

### Preparation 8

| N° | Description | % (w/w) a |
|---|---|---|
| 01 | Dimethyl isosorbide | 75.00 |
| 02 | Salicylic acid | 15.00 |
| 03 | Dimethyl sulphone | 10.00 |

Method of preparation: dissolve 03 in 01, to the solution obtained, mix in 02

### Preparation 9 as comparative example

| N° | Description | % (w/w) a |
|---|---|---|
| 01 | Dimethyl isosorbide | 20.00 |
| 02 | Tartaric acid | 30.00 |
| 03 | Propylene glycol | 15.00 |
| 04 | Water | 35.00 |

Method of preparation: dissolve 02 in 01, to the solution obtained, mix in 03+04

### Preparation 10 as comparative example

| N° | Description | % (w/w) a |
|---|---|---|
| 01 | Dimethyl isosorbide | 30.00 |
| 02 | Glycolic acid | 60.00 |
| 03 | Water | 10.00 |

Method of preparation: dissolve 02 in 01. Mi the solution obtained with 03

### Preparation 11

| N° | Description | % (w/w) a |
|---|---|---|
| 01 | Dimethyl isosorbide | 30.00 |
| 02 | Glycolic acid | 50.00 |
| 03 | dimethyl sulfone | 5.00 |
| 04 | Water | 15.00 |

Method of preparation: dissolve 02 + 03 in 01. Mix the solution obtained in 02

### Preparation 12 as comparative example

| N° | Description | % (w/w) a |
|---|---|---|
| 01 | Dimethyl isosorbide | 50.00 |
| 02 | Resorcine | 10.00 |
| 03 | Salicylic acid | 20.00 |
| 04 | Ethyl alcohol | 20.00 |

Method of preparation: dissolve 02 + 03 in 04; to the solution obtained mix in 01

### Preparation 13 as comparative example

| N° | Description | % (w/w) a |
|---|---|---|
| 01 | Dimethyl isosorbide | 60.00 |
| 02 | Resorcine | 10.00 |
| 03 | Salicylic acid | 20.00 |
| 05 | Ethyl alcohol | 10.00 |

Method of preparation: dissolve 02 + 03 in 05; to the solution obtained mix in 01

### Preparation 14

| N° | Description | % (w/w) a |
|---|---|---|
| 01 | Dimethyl isosorbide | 50.00 |
| 02 | Retinoic acid | 1.00 |
| 03 | Salicylic acid | 20.00 |
| 04 | Dimethyl sulphone | 20.00 |
| 05 | Ethyl alcohol | 9.00 |

Method of preparation: mix 01 + 05; dissolve 02 + 03 + 04 in the solution obtained

### Preparation 15

| N° | Description | % (w/w) a |
|---|---|---|
| 01 | Dimethyl isosorbide | 20.00 |
| 02 | Glycolic acid | 50.00 |
| 03 | Dimethyl sulphone | 10.00 |
| 04 | Ethyl lactate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

### Preparation 16

| N° | Description | % (w/w) a |
|---|---|---|
| 01 | Dimethyl isosorbide | 30.00 |
| 02 | Lactic acid | 40.00 |
| 03 | Dimethyl sulphone | 10.00 |
| 04 | Ethyl lactate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

### Preparation 17

| N° | Description | % (w/w) a |
|---|---|---|
| 01 | Dimethyl isosorbide | 50.00 |
| 02 | Trichloroacetic acid | 20.00 |
| 03 | Dimethyl sulphone | 10.00 |
| 04 | Ethyl pyruvate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

### Preparation 18

| N° | Description | % (w/w) a |
|---|---|---|
| 01 | Dimethyl isosorbide | 50.00 |
| 02 | Salicylic acid | 20.00 |
| 03 | Dimethyl sulphone | 10.00 |
| 04 | Ethyl pyruvate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

### Preparation 19

| N° | Description | % (w/w) a |
|---|---|---|
| | PHASE A | |
| 01 | Steareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl pyruvate | 5.00 |
| | PHASE B | |
| 08 | Propylene glycol | 2.00 |
| 09 | Pyruvic acid | 10.00 |
| 10 | Demineralised water | 10.00 |
| | PHASE C | |
| 11 | Dimethyl sulphone | 10.00 |
| 12 | Propylene glycol | 2.00 |
| 13 | Disodium EDTA | 0.07 |
| 14 | Glycerol | 5.00 |
| 15 | Phenoxyethanol | 1.00 |
| 16 | Methyl paraben | 0.10 |
| 17 | Ethyl paraben | 0.10 |
| 18 | Propyl paraben | 0.10 |
| 19 | Demineralised water qba | 100 |

Method of preparation: heat PHASE A) to 75°C; heat PHASE C to +75°C; combine PHASE A with PHASE C with stirring homogenising the solution; cool to +45°C; then combine with PHASE B still with stirring and cool to 25°C.

### Preparation 20

| N° | Description | % (w/w) a |
|---|---|---|
| | PHASE A | |
| 01 | Steareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl lactate | 5.00 |
| | PHASE B | |
| 08 | Propylene glycol | 2.00 |
| 09 | Tartaric acid | 15.00 |
| 10 | Demineralised water | 10.00 |
| | PHASE C | |
| 11 | Dimethyl sulphone | 10.00 |
| 12 | Propylene glycol | 2.00 |
| 13 | Disodium EDTA | 0.07 |
| 14 | Glycerol | 5.00 |
| 15 | Phenoxyethanol | 1.00 |
| 16 | Methyl paraben | 0.10 |
| 17 | Ethyl paraben | 0.10 |
| 18 | Propyl paraben | 0.10 |
| 19 | Demineralised water qba | 100 |

Method of preparation: heat PHASE A) to 75°C; heat PHASE C to +75°C; combine PHASE A with PHASE C with stirring homogenising the solution; cool to +45°C; then combine with PHASE B still with stirring and cool to 25°C.

### Preparation 21

| N° | Description | % (w/w) a |
|---|---|---|
| | PHASE A | |
| 01 | Steareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Zinc oxide oily solution 50% | 20.00 |
| | PHASE B | |
| 08 | Propylene glycol | 2.00 |
| 09 | Lactic acid | 10.00 |
| 10 | Demineralised water | 10.00 |
| | PHASE C | |
| 11 | Dimethyl sulphone | 10.00 |
| 12 | Propylene glycol | 2.00 |
| 13 | Disodium EDTA | 0.07 |
| 14 | Glycerol | 5.00 |
| 15 | Phenoxyethanol | 1.00 |
| 16 | Methyl paraben | 0.10 |
| 17 | Ethyl paraben | 0.10 |
| 18 | Propyl paraben | 0.10 |
| 19 | Demineralised water qba | 100 |

Method of preparation: heat PHASE A) to 75°C; heat PHASE C to +75°C; combine PHASE A with PHASE C with stirring homogenising the solution; cool to +45°C; then combine with PHASE B still with stirring and cool to 25°C.

In the present description, the mixture of dimethyl isosorbide, associated with a keratolytic agent and/or a mixture of keratolytic agents, together with dimethyl sulphone, can be combined with esters of the keratolytic agents, preferably the ethyl esters. The use of the keratolytic agent esters, singularly and/or in association, is justified by the fact that, once absorbed into the cuteous, these are hydrolysed liberating the acid and alcohol. The acid form will therefore be able to continue the keratolytic action, in a less intense form but with a more protracted effect over time. Examples of the esters of the keratolytic agents are ethyl pyruvate, ethyl glycolate, triethyl citrate, ethyl resorcinate, the ethyl ester of retinoic acid, ethyl salicylate, methyl salicylate, ethyl malnate, ethyl acetate, ethyl tartrate.

A further subject of the present invention is a formulation for chemical peeling comprising of one or more keratolytic agents, preferably selected from the above described group, together with a keratolytic agent ester. The ester of the keratolytic agent will preferably be selected from the above listed group and can be the ester of the same keratolytic agent used in acid form or the ester of a different keratolytic agent. Such keratolytic agent esters will be present in the composition in a quantity preferably comprised of between 3% and 60% by weight, more preferably in a quantity comprised of between 15% and 50% by weight, with respect to the keratolytic agent (or to the mixture of keratolytic agents).

In place of the keratolytic agent esters, another derivative or pro-drug can however be used, in the same proportions indicated above, which is capable of liberating after administration under biological conditions, the keratolytic agent in the treatment site.

For comparative purpose, it is evident that the combination of one or more keratolytic agents and their derivative or pro-drug as defined above can also be applied to compositions in which the dimethyl isosorbide or the dimethyl sulphone are not present. One will in fact obtain, in any case, the desired effect of achieving a more prolonged over time and at the same time less acute chemical peeling, with the consequent reduction of the irritant phenomena caused by intense and acute treatment.

## Claims

1. Dimethyl sulphone combined with a keratolytic agent or a mixture of keratolytic agents for use as anti-inflammatory agent against the inflammation produced by a keratolytic agent or a mixture of keratolytic agents, wherein dimethyl sulphone is present in a quantity comprised between 2% and 70% by weight, preferably between 10% and 60% by weight with respect to the keratolytic agent or the mixture of keratolytic agents.

2. Dimethyl sulphone combined with a keratolytic agent or a mixture of keratolytic agents for use according to claim 1, wherein said keratolytic agent is selected from the group comprising saturated and unsaturated monocarboxylic acids, saturated and unsaturated bicarboxylic acids, tricarboxylic acids, alpha hydroxyacids and beta hydroxyacids of monocarboxylic acids, alpha hydroxyacids and beta hydroxyacids of bicarboxylic acids, alpha hydroxyacids and beta hydroxyacids of tricarboxylic acids, ketoacids, alpha ketoacids, beta ketoacids, polycarboxylic acids, polyhydroxy monocarboxylic acids, polyhydroxy bicarboxylic acids, polyhydroxy tricarboxylic acids.

3. Dimethyl sulphone combined with a keratolytic agent or a mixture of keratolytic agents for use according to claim 1 or 2, wherein said keratolytic agent is selected from the group comprising glycolic acid, tartaric acid, salicylic acid, citric acid, lactic acid, pyruvic acid, gluconic acid, glucuronic acid, malic acid, oxalic acid, malonic acid, succinic acid, acetic acid, phenol, resorcine, retinoic acid, adapalene, trichloroacetic acid, 5-fluoro uracil, azelaic acid.

4. Dimethyl sulphone combined with a keratolytic agent or a mixture of keratolytic agents for use according to claim 3, wherein said keratolytic agent is a salt, an ester, a cis or trans form, a racemic mixture and/or a relative dextrorotatory or levorotatory form of said keratolytic agent.

5. Dimethyl sulphone combined with a keratolytic agent or a mixture of keratolytic agents for use according to claim 4, wherein said ester is an ethyl ester.

6. Dimethyl sulphone combined with a keratolytic agent or a mixture of keratolytic agents for use according to any one of the preceding claims, comprising a keratolytic agent according to claim 3 and an ester of said keratolytic agent.

7. Dimethyl sulphone combined with a keratolytic agent or a mixture of keratolytic agents for use according to claim 6, wherein the percentage of ester of the keratolytic agent is comprised between 3% and 60% by weight, preferably between 15% and 50% by weight with respect to the keratolytic agent.

## Patentansprüche

1. Dimethylsulfon, kombiniert mit einem Keratolytikum oder einer Mischung von Keratolytika zur Verwendung als entzündungshemmendes Mittel gegen die Entzündung, erzeugt durch ein Keratolytikum oder eine Mischung von Keratolytika, wobei Dimethylsulfon in einer Menge vorhanden ist, die zwischen 2 Gewichts-% und 70 Gewichts-%, bevorzugt zwischen 10 Gewichts-% und 60 Gewichts-%, in Bezug auf das Keratolytikum oder die Mischung von Keratolytika umfasst ist.

2. Dimethylsulfon, kombiniert mit einem Keratolytikum oder einer Mischung von Keratolytika zur Verwendung nach Anspruch 1,
wobei das Keratolytikum aus der Gruppe ausgewählt ist, umfassend gesättigte und ungesättigte Monocarboxylsäuren, gesättigte und ungesättigte Dicarboxylsäuren, Tricarboxylsäuren, alpha-Hydroxysäuren und beta-Hydroxysäuren von Monocarboxylsäuren, alpha-Hydroxysäuren und beta-Hydroxysäuren von Dicarboxylsäuren, alpha-Hydroxysäuren und beta-Hydroxysäuren von Tricarboxylsäuren, Ketosäuren, alpha-Ketosäuren, beta-Ketosäuren, Polycarboxylsäuren, Polyhydroxymonocarboxylsäuren, Polyhydroxydicarboxylsäuren, Polyhydroxytricarboxylsäuren.

3. Dimethylsulfon, kombiniert mit einem Keratolytikum oder einer Mischung von Keratolytika zur Verwendung nach Anspruch 1 oder 2,
wobei das Keratolytikum aus der Gruppe ausgewählt ist, umfassend Glykolsäure, Weinsäure, Salizylsäure, Zitronensäure, Milchsäure, Benztraubensäure, Gluconsäure, Glucuronsäure, Apfelsäure, Oxalsäure, Malonsäure, Bernsteinsäure, Essigsäure, Phenol, Resorcin, Retinsäure, Adapalen, Trichloressigsäure, 5-Fluoruracil, Azelainsäure.

4. Dimethylsulfon, kombiniert mit einem Keratolytikum oder einer Mischung von Keratolytika zur Verwendung nach Anspruch 3,
wobei das Keratolytikum ein Salz, ein Ester, eine cis- oder eine trans-Form, eine razemische Mischung und/oder eine relativ rechtsdrehende oder linksdrehende Form von dem Keratolytikum ist.

5. Dimethylsulfon, kombiniert mit einem Keratolytikum oder einer Mischung von Keratolytika zur Verwendung nach Anspruch 4,
wobei der Ester ein Ethylester ist.

6. Dimethylsulfon, kombiniert mit einem Keratolytikum oder einer Mischung von Keratolytika zur Verwendung nach einem der vorhergehenden Ansprüche, umfassend ein Keratolytikum nach Anspruch 3 und einen Ester des Keratolytikums.

7. Dimethylsulfon, kombiniert mit einem Keratolytikum oder einer Mischung von Keratolytika zur Verwendung nach Anspruch 6,
wobei der Prozentsatz an Ester des Keratolytikums zwischen 3 Gewichts-% und 60 Gewichts-%, bevorzugt zwischen 15 Gewichts-% und 50 Gewichts-%, in Bezug auf das Keratolytikum umfasst ist.

## Revendications

1. Diméthylsulfone combinée avec un agent kératolytique ou un mélange d'agents kératolytiques pour une utilisation en tant qu'agent anti-inflammatoire contre l'inflammation produite par un agent kératolytique ou un mélange d'agents kératolytiques, dans laquelle la diméthylsulfone est présente dans une quantité comprise entre 2 % et 70 % en poids, de préférence entre 10 % et 60 % en poids par rapport à l'agent kératolytique ou au mélange d'agents kératolytiques.

2. Diméthylsulfone combinée avec un agent kératolytique ou un mélange d'agents kératolytiques pour une utilisation selon la revendication 1, dans laquelle ledit agent kératolytique est choisi dans le groupe comprenant des acides monocarboxyliques saturés et insaturés, des acides bicarboxyliques saturés et insaturés, des acides tricarboxyliques, des acides alpha-hydroxylés et des acides bêta-hydroxylés d'acides monocarboxyliques, des acides alpha-hydroxylés et des acides bêta-hydroxylés d'acides bicarboxyliques, des acides alpha-hydroxylés et des acides bêta-hydroxylés d'acides tricarboxyliques, des acides cétoniques, des acides alpha-cétoniques, des acides bêta-cétoniques, des acides polycarboxyliques, des acides monocarboxyliques polyhydroxylés, des acides bicarboxyliques polyhydroxylés, des acides tricarboxyliques polyhydroxylés.

3. Diméthylsulfone combinée avec un agent kératolytique ou un mélange d'agents kératolytiques pour une utilisation selon la revendication 1 ou la revendication 2, dans laquelle ledit agent kératolytique est choisi dans le groupe comprenant l'acide glycolique, l'acide tartrique, l'acide salicylique, l'acide citrique, l'acide lactique, l'acide pyruvique, l'acide gluconique, l'acide glucuronique, l'acide malique, l'acide oxalique, l'acide malonique, l'acide succinique, l'acide acétique, le phénol, la résorcinol, l'acide rétinoïque, l'adapalène, l'acide trichloroacétique, le 5-fluoro-uracile, l'acide azélaïque.

4. Diméthylsulfone combinée avec un agent kératolytique ou un mélange d'agents kératolytiques pour une utilisation selon la revendication 3, dans laquelle ledit agent kératolytique est un sel, un ester ou une forme cis ou trans, un mélange racémique et/ou une forme dextrogyre ou lévogyre relative dudit agent kératolytique.

5. Diméthylsulfone combinée avec un agent kératolytique ou un mélange d'agents kératolytiques pour une utilisation selon la revendication 4, dans laquelle ledit ester est un ester d'éthyle.

6. Diméthylsulfone combinée avec un agent kératolytique ou un mélange d'agents kératolytiques pour une utilisation selon l'une quelconque des revendications précédentes, comprenant un agent kératolytique selon la revendication 3 et un ester dudit agent kératolytique.

7. Diméthylsulfone combinée avec un agent kératolytique ou un mélange d'agents kératolytiques pour une utilisation selon la revendication 6, dans laquelle le pourcentage d'ester de l'agent kératolytique est compris entre 3 % et 60 % en poids, de préférence entre 15 % et 50 % en poids par rapport à l'agent kératolytique.
